# EUROPEAN PATENT SPECIFICATION

(11) **EP 0 761 249 B1**
(45) Date of publication and mention of the grant of the patent: **01.10.2003**
(21) Application number: 96112317.1
(22) Date of filing: 30.07.1996
(51) Int. Cl.: A61M 16/01, A61M 16/18

(54) **Anaesthetic apparatus**
Anästhesiegerät
Appareil d'anesthésie

(30) Priority: 12.09.1995 SE 9503141
(43) Date of publication of application: 12.03.1997
(73) Proprietor: Siemens-Elema AB, 171 95 Solna (SE)
(72) Inventor: Psaros, Georgios, 146 38 Tullinge (SE); Castor, Rolf, 129 42 Hägersten (SE)

(56) References cited:
- EP-A- 0 243 259
- EP-A- 0 692 273
- WO-A-95/01137
- GB-A- 2 007 508
- SE-B- 430 213
- US-A- 4 611 590
- US-A- 5 080 093
- US-A- 5 287 849

## Description

The present invention relates to an anaesthetic apparatus according to the preamble to claim 1.

SE-B-430 213 describes a respirator comprising a respiratory circuit with an inspiratory line, an expiratory line and a tracheal tube for connecting the respirator to a patient. Gas can be carried from a first source of gas in the respirator to the patient in the inspiratory line and tracheal tube. Gas can also be supplied from a second source of gas, directly to the tracheal tube, through a separate gas line. An anaesthetic gas can inter alia be carried directly to the connecting line. Gas can be supplied continuously or according to a specific pattern, e.g. during an initial part of an inspiration. Gas flows are regulated with valves controlled by a control device.

However, regulating these valves can be rather complicated, since the control of gases and gas flows is involved. All the lines create flow resistances and compressible spaces. Gas flows from two lines (the inspiratory line and the separate gas line) must be co-ordinated and even made to mix. Generally speaking, a simpler, more reliable and more easily controlled anaesthetic apparatus for supplying anaesthetic gas to a living creature would be desirable.

One object of the invention is to achieve an anaesthetic apparatus which eliminates the described problems.

This object is achieved in accordance with the invention in that the anaesthetic apparatus, according to the preamble, is devised according to the characterizing portion to claim 1.

Instead of supplying gaseous anaesthetic to the respiratory circuit, the anaesthetic is supplied in droplets by means of a micropump. In other words, it is supplied in the form of microdroplets, preferably less than 100 nm in diameter. This makes it possible for supply to take place inside the connection means, i.e. very close to the patient. The tiny microdroplets are rapidly vaporized in the connection means. The concentration of anaesthetic can be varied rapidly and reliably when anaesthetic is supplied. No additional gas lines, with attendant resistances to flow and compressibility, are needed.

Advantageous improvements of the anaesthetic apparatus according to the invention are set forth in the dependent claims.

The micropump appropriately consists of a piezoelectric, pump. Such pumps are well-known in the ink jet art and do not require any detailed description. The liquid anaesthetic's physical properties, such as its molecular weight, vapour pressure, surface tension and heat of vaporization, are programmed into the micropump's control unit. The size of droplets can then be regulated with extreme accuracy. So dispensing is accordingly very exact.

In the same way as in the prior art, anaesthetic can be supplied during part of a breathing cycle, e.g. during an initial part of the inspiratory phase.

In one embodiment of the anaesthetic apparatus, the supply of anaesthetic can be regulated based on the measured momentary flow of respiratory gas and a reference value for the end concentration of anaesthetic. Control can be refined by e.g. taking the temperature and pressure of the respiratory gas and the temperature of the liquid anaesthetic into account. These parameters can be measured and/or regulated to achieve predetermined values. The end concentration can also be controlled by a concentration meter.

To facilitate vaporization of the droplets of liquid anaesthetic, a vaporizer means can be located in the connection means in such a way that droplets from the micropump fall onto this vaporization means and are evaporated. The temperature of the vaporization means can be regulated according to the liquid anaesthetic's vaporization characteristics.

A plurality of containers holding different liquid anaesthetics can be arranged in parallel in the dosing unit and connected to the micropump via a valve unit which only permits the release of liquid anaesthetic from one container at a time. This makes it possible to switch anaesthetic agents rapidly and achieve a dosing unit which is much smaller than known anaesthetic vaporizers.

Embodiments of the anaesthetic apparatus according to the invention will now be described in greater detail, referring to the figures in which
FIG. 1 shows a first embodiment of the anaesthetic apparatus according to the invention;
FIG. 2 shows a dosing unit in the anaesthetic apparatus; and
FIG. 3 shows a second embodiment of the anaesthetic apparatus according to the invention.

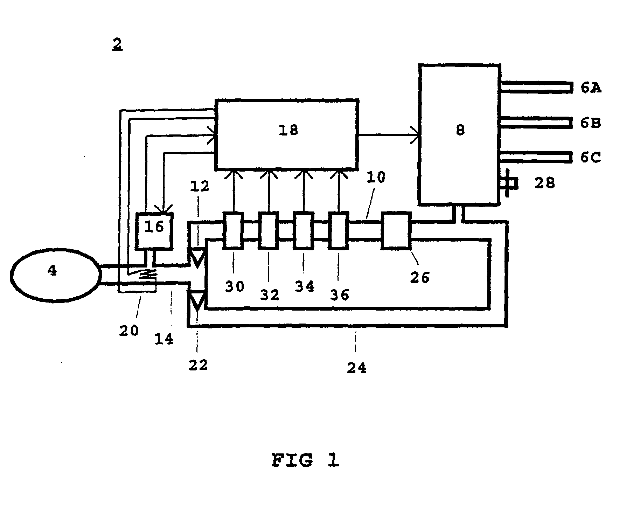

The anaesthetic apparatus 2 in FIG. 1 is connected to a patient 4 in order to supply her/him with a respiratory gas and an anaesthetic. The respiratory gas is prepared by connecting the anaesthetic apparatus 2 to a first gas connection 6A, a second gas connection 6B and a third gas connection 6C. The components of the respiratory gas, such as oxygen (O₂) and nitrous oxide (N₂O), are carried via the gas connections to a gas regulation unit 8. The supplied gas components are mixed in specific proportions in the gas regulation unit 8.

The patient's 4 respiration rhythm (respiratory depth and respiration rate) is also regulated via the gas regulation unit 8. Thus, respiratory gas is supplied to the patient 4 through an inspiratory line 10 and a connection means 14. A first check valve 12 is arranged in the inspiratory line 10 to control the direction of flow of the respiratory gas. The connection means 14 can consist of a tracheal tube or the equivalent.

A dosing unit 16 is connected to the connection means 14 in order to supply anaesthetic in the form of liquid droplets. The dosing unit 16, which will be described below in greater detail, is controlled by a control unit 18, which also controls the gas regulation unit 8. The dosing unit 16 pumps out anaesthetic in droplet form onto a vaporizer means 20 to facilitate the anaesthetic's vaporization before it is carried with respiratory gas down into the patient's 4 lungs.

The temperature of the vaporization means 20 can be regulated by the control unit 18.

In expiration, respiratory gas is carried through an expiratory line 24 back to the gas regulation unit 8. A second check valve 22 is arranged in the expiratory line 24 to control the direction of flow.

The anaesthetic apparatus 2 operates as a closed circuit system. This means that as much of the respiratory gas as possible is re-used after expiration. A carbon dioxide absorber 26 is therefore arranged in the inspiratory line 10 to absorb carbon dioxide expired by the patient 4. A pressure relief valve 28 is also arranged in the gas regulation unit 8 to keep the pressure of gas in the respiratory circuit from exceeding a predefined value (surplus gas is bled off by the pressure relief valve 28 if that predefined pressure is reached). Since the system is closed, a minimal amount of fresh gas is supplied through the gas connections 6A, 6B and 6C during surgery. However, the flow of fresh respiratory gas is much greater during the induction of narcosis and recovery from same.

Control of the supply of respiratory gas is refined by the measurement of a plurality of parameters for respiratory gas in the inspiratory line 10 and taken into account in the control unit's 18 dispensing. So temperature is measured in a first thermometer 30, pressure in a pressure meter 32, momentary flow in a flow meter 34 and concentration in a gas meter 36.

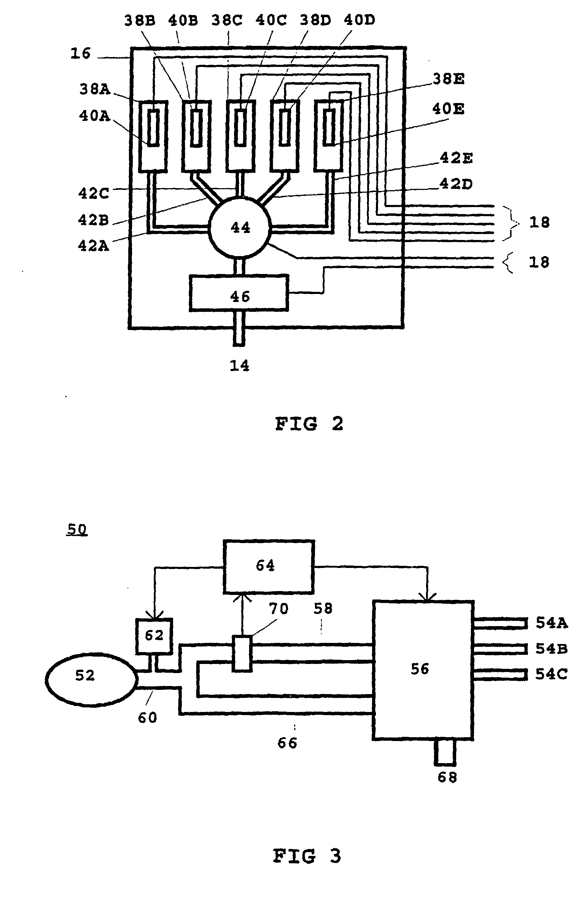

Dispensing will now be described in greater detail, referring to the dosing unit 16 in FIG. 2. The dosing unit 16 comprises a first container 38A for a first anaesthetic, a second container 38B for a second anaesthetic, a third container 38C for a third anaesthetic, a fourth container 38D for a fourth anaesthetic and a fifth container 38E for a fifth anaesthetic. The anaesthetics are in liquid form and consist appropriately of halothane, enflurane, desflurane, isoflurane and sevoflurane.

A second thermometer 40A is arranged in the first container 38A to measure the temperature of the first liquid anaesthetic, a third thermometer 40B is arranged in the second container 38B to measure the temperature of the second liquid anaesthetic, a fourth thermometer 40C is arranged in the third container 38C to measure the temperature of the third liquid anaesthetic, a fifth thermometer 40D is arranged in the fourth container 38D to measure the temperature of the fourth liquid anaesthetic and a sixth thermometer 40E is arranged in the fifth container 38E to measure the temperature of the fifth liquid anaesthetic. Measured temperatures are transferred to the control unit 18. The thermometers 40A, 40B, 40C, 40D, 40E can also consist of temperature regulators for regulating the temperatures of the liquid anaesthetic at the predefined temperature for each liquid anaesthetic.

A first liquid line 42A from the first liquid container 38A leads to a valve unit 44, a second liquid line 42B from the second container 38B leads to the valve unit 44, a third liquid line 42C from the third container 38C leads to the valve unit 44, a fourth liquid line 42D from the fourth container 38D leads to the valve unit 44 and a fifth liquid line 42E from the fifth container 38E leads to the valve unit 44. The valve unit 44 is devised so only one liquid line 42A, 42B, 42C, 42D, 42E at a time can release liquid to a micropump 46. The micropump 46 pumps out liquid anaesthetic in the form of microdroplets, preferably less than 100 nm in diameter. Both the valve unit 44 and the micropump 46 are controlled by the control unit 18. The micropump 46 is preferably a piezoelectric pump.

FIG. 3 shows a second embodiment of the anaesthetic apparatus, designated 50, according to the invention. The anaesthetic apparatus 50, which is connected to a patient 52, operates as an open system, i.e. no gas is re-used. The gas components in the respiratory gas are sent to the anaesthetic apparatus 50 through a first gas connection 54A, a second gas connection 54B and a third gas connection 54C. Like the previous embodiment, the gas components can consist of O₂ and N₂O. The gas components are mixed in specific proportions in a gas regulation unit 56 which also regulates the pressure and flow of respiratory gas supplied to the patient 52.

Here, the respiratory gas passes through an inspiratory line 58 and a connection means 60 to the patient 52. A dosing unit 62 is connected to the connection means 60 to supply it with anaesthetic in droplet form. The droplets are so small and the flow of respiratory gas is so fast that the droplets vaporize before they reach the patient's 52 lungs. Anaesthetic is only supplied during part of each inspiratory phase, since the relative concentration in the lungs is sufficient to sustain the desired depth of anaesthesia in the patient 52. The consumption of anaesthetic is therefore considerably lower than in other known open systems in which anaesthetic gas is mixed with respiratory gas at an earlier stage.

In expiration, respiratory gas passes through an expiratory line 66 before it returns to the gas regulation unit 56 for discharge through the evacuation line 68 into some form of gas collection vessel or filter device.

The dispensing of anaesthetic droplets is regulated by a control unit 64 according to the value measured for the momentary flow of respiratory gas in the inspiratory line 58. Flow is measured in a flowmeter 70. The control unit also controls the gas regulation unit 56.

The anaesthetic apparatus according to the invention can also be devised for semi-open or semi-closed systems, or combinations of such systems and the described embodiments. Instead of a dosing unit with a plurality of containers, a plurality of dosing units, each of which with one or more containers, can be connected to the connection means.

## Claims

1. An anaesthetic apparatus (2; 50), comprising a respiratory circuit (10, 14, 24; 58, 60, 66), through which a respiratory gas containing a predefined amount of an anaesthetic is supplied to a living creature (2; 52) during an inspiratory phase and through which the respiratory gas is removed from the living creature (2;52) during an expiratory phase, and a dosing unit (16; 62) for supplying the anaesthetic to a connection means (14; 60) in the respiratory circuit (10, 14, 24; 58, 60, 66), said connection means (14; 60) being directly connectable to the living creature (2; 52) whereby respiratory gas will flow through the connection means (14;60) during both inspiratory and expiratory phases, **characterized in that** the dosing unit (16; 62) is arranged to supply liquid anaesthetic to the connection means (14; 60), and the dosing unit (16; 62) comprises a container (38A) holding liquid anaesthetic and a micropump (46) devised to supply liquid anaesthetic in microdroplets, preferably less than 100 nm in diameter, to the connection means (14; 60), the micropump (46) being controlled by a control unit (18; 64), and the essential physical properties of the liquid anaesthetic used are programmed into the control unit (18;64).

2. An anaesthetic apparatus according to claim 1, **characterized in that** the micropump (46) is a piezoelectric pump.

3. An anaesthetic apparatus of claim 1 or 2, **characterized in that** the control unit (18; 64) is arranged to regulate the micropump's (46) release of anaesthetic in such a way that the micropump (46) only releases liquid anaesthetic during part of the inspiratory phase.

4. An anaesthetic apparatus according to any of the above claims, **characterized by** a flowmeter (34; 70), arranged in an inspiratory line (10; 58) in the respiratory circuit (10, 14, 24; 58, 60, 66) to determine a value for the momentary flow of respiratory gas, the control unit (18; 64) regulating the micropump's (46) release of liquid anaesthetic according to the momentary flow value determined by the flowmeter (34; 70) and a predefined reference value, corresponding to the desired concentration of anaesthetic in the respiratory gas.

5. An anaesthetic apparatus according to any of the above claims, **characterized by** a first thermometer (30), arranged in the respiratory circuit (10, 14, 24) to measure the temperature of the respiratory gas, a second thermometer (40A) is arranged in the container (38A) to measure the temperature of the liquid anaesthetic and a pressure meter (32) is arranged in the respiratory circuit (10, 14, 24) to measure the pressure of respiratory gas, the control unit (18) then controlling the micropump's (46) release of liquid anaesthetic according to measured temperatures and pressure.

6. An anaesthetic apparatus of any of the above claims, **characterized by** a heatable vaporizer means (20), arranged in the connection means (14) to vaporize liquid anaesthetic from the dosing unit (16).

7. An anaesthetic apparatus according to claim 6, **characterized in that** the control unit (18) is connected to the vaporizing means (20) to regulate the temperature of the means, the temperature then depending on the liquid anaesthetic's vaporization properties.

8. An anaesthetic apparatus according to any of the above claims, **characterized in that** the dosing unit (16) comprises at least one additional container (38B, 38C, 38D, 38E) holding an additional liquid anaesthetic, and the container (38A) and the additional container (38B, 38C, 38D, 38E) are connected to the micropump (46) via a valve unit (14) devised so only one container (38A, 38B, 38C, 38D, 38E) at a time can release liquid anaesthetic to the micropump (46).

9. An anaesthetic apparatus according to any of the above claims, **characterized by** a gas meter (36), arranged in the respiratory circuit (10, 14, 24) to measure the concentration of anaesthetic in the respiratory gas, the control unit (18) then controlling the micropump (46) according to the measured concentration of the anaesthetic.

## Patentansprüche

1. Ein Anästhesiegerät (2; 50) mit einem Atemkreis (10, 14, 24; 58, 60, 66), über den ein Atemgas, das eine vorbestimmte Menge Narkosemittel enthält, einem Lebewesen (2; 52) während einer Inspirationsphase zugeführt wird, und über den das Atemgas während der Exspirationsphase von dem Lebewesen (2; 52) entfernt wird, und einer Dosiereinheit (16; 62) zum Zuführen des Narkosemittels zu einem Verbindungsmittel (14; 60) in dem Atemkreis (10, 14, 24; 58, 60, 66), wobei das Verbindungsmittel (14; 60) direkt mit dem Lebewesen (2; 52) verbindbar ist, wodurch Atemgas sowohl während der Inspirations- als auch während der Exspirationsphase durch das Verbindungsmittel (14; 60) fließen wird, **dadurch gekennzeichnet, dass** das Dosiermittel(16; 62) angeordnet ist, flüssiges Narkosemittel dem Verbindungsmittel (14; 60) zuzuführen, und das Dosiermittel (16; 62) einen flüssiges Narkosemittel enthaltenden Behälter (38A) und eine Mikropumpe (46), die ausgebildet ist, flüssiges Narkosemittel in Mikrotropfen, vorzugsweise kleiner als 100 nm im Durchmesser, dem Verbindungsmittel (14; 60) zuzuführen, aufweist, wobei die Mikropumpe (46) über eine Steuereinheit (18; 64) gesteuert wird und wesentliche physikalische Größen des verwendeten flüssigen Narkosemittels in die Steuereinheit (18; 64) programmiert werden.

2. Ein Anästhesiegerät gemäß Anspruch 1, **dadurch gekennzeichnet, dass** die Mikropumpe (46) eine piezoelektrische Pumpe ist.

3. Ein Anästhesiegerät gemäß Anspruch 1 oder 2, **dadurch gekennzeichnet, dass** die Steuereinheit (18; 64) ausgebildet ist, um die Narkosemittelabgabe der Mikropumpe (46) so zu regeln, dass die Mikropumpe (46) flüssiges Narkosemittel nur währende eines Teils der Inspirationsphase abgibt.

4. Ein Anästhesiegerät gemäß einem der obigen Ansprüche, **gekennzeichnet durch** einen in der Inspirationsleitung (10; 58) des Atemkreises (10, 14, 24; 58, 60 66) angeordneten Flussmesser (34; 70) zum Bestimmen des Wertes des aktuellen Atemgasflusses, wobei die Steuereinheit (18; 64) die Abgabe flüssigen Narkosemittels **durch** die Mikropumpe (46) in Abhängigkeit von dem aktuellen, **durch** den Flussmesser (34; 70) bestimmen Flusswert und einem vorbestimmen Referenzwert, der der gewünschten Konzentration von Narkosemittel in dem Atemgas entspricht, steuert.

5. Ein Anästhesiegerät gemäß einem der obigen Ansprüche, **gekennzeichnet durch** ein erstes, in dem Atemkreis (10, 14, 24) angeordnetes Thermometer (30) zum Messen der Temperatur des Atemgases, ein zweites, in dem Behälter (38A) angeordnetes Thermometer (40A) zum Messen der Temperatur des flüssigen Narkosemittels und einem in dem Atemkreis (10, 14, 24) angeordneten Druckmesser (32) zum Messen des Atemgasdruckes, wobei die Steuereinheit (18) dann die Abgabe flüssigen Narkosemittels **durch** die Mikropumpe (46) in Abhängigkeit von den gemessenen Temperaturen und dem gemessenen Druck steuert.

6. Ein Anästhesiegerät nach einem der obigen Ansprüche, **gekennzeichnet durch** ein heizbares Verdampfermittel (20), das in dem Verbindungsmittel (14) angeordnet ist, um flüssiges Narkosemittel aus der Dosiereinheit (16) zu verdampfen.

7. Ein Anästhesiegerät gemäß Anspruch 6, **dadurch gekennzeichnet, dass** die Steuereinheit (18) mit dem Verdampfermittel (20) verbunden ist, um die Temperatur des Mittels zu regeln, wodurch die Temperatur dann von den Verdampfungseigenschaften des flüssigen Narkosemittels abhängt.

8. Ein Anästhesiegerät nach einem der obigen Ansprüche, **dadurch gekennzeichnet, dass** die Dosiereinheit (16) zumindest einen zusätzlichen Behälter (38B, 38C, 38D, 38E) aufweist, der ein zusätzliches Narkosemittel enthält, und dass der Behälter (38A) und der zusätzliche Behälter (38B, 38C, 38D, 38E) mit der Mikropumpe (46) über eine Ventileinheit (14) verbunden sind, die so ausgebildet ist, dass jeweils nur ein Behälter (38A, 38B, 38C, 38D, 38E) flüssiges Narkosemittel an die Mikropumpe (46) abgeben kann.

9. Ein Anästhesiegerät nach einem der obigen Ansprüche, **gekennzeichnet durch** einen in dem Atemkreis (10, 14, 24) angeordneten Gasmesser (36) zum Messen der Konzentration des Narkosemittels in dem Atemgas, wobei die Steuereinheit (18) dann die Mikropumpe (46) gemäß der gemessenen Narkosemittelkonzentration steuert.

## Revendications

1. Dispositif (2 ; 50) d'anesthésie comprenant un circuit (10, 14, 24 ; 58, 60, 66) de respiration, par lequel un gaz de respiration contenant une quantité définie à l'avance d'un produit anesthésique est envoyé à une créature (2 ; 52) vivante pendant une phase (1) d'inspiration et par lequel le gaz de respiration est éliminé de la créature (2 ; 52) vivante pendant une phase d'expiration, et une unité (16 ; 62) de dosage destinée à envoyer le produit anesthésique à un moyen (14 ; 60) de liaison du circuit (10, 14, 24 ; 58, 60, 66) de respiration, le moyen (14 ; 60) de liaison pouvant être relié directement à la créature (2 ; 52) vivante, de sorte que du gaz de respiration passe dans le moyen de liaison (14 ; 60) à la fois pendant les phases d'inspiration et d'expiration, **caractérisé en ce que** l'unité (16 ; 62) de dosage est conçue pour envoyer du produit anesthésique liquide au moyen (14 ; 60) de liaison, et l'unité (16 ; 62) de dosage comprend un récipient (38A) contenant du produit anesthésique liquide et une micropompe (46) destinée à envoyer du produit anesthésique liquide en gouttelettes microscopiques d'un diamètre de préférence inférieur à 100 nm au moyen (14 ; 60) de liaison, la micropompe (46) étant commandée par une unité (18 ; 64) de commande, et les propriétés physiques essentielles du produit anesthésique liquide utilisé étant programmées dans l'unité (18 ; 64) de commande.

2. Dispositif d'anesthésie suivant la revendication 1, **caractérisé en ce que** la micropompe (46) est une pompe piézoélectrique.

3. Dispositif d'anesthésie suivant la revendication 1 ou 2, **caractérisé en ce que** l'unité (18 ; 64) de commande est conçue pour réguler le refoulement du produit anesthésique par la micropompe (46) d'une façon telle que la micropompe (46) ne refoule du produit anesthésique liquide que pendant une partie de la phase d'inspiration.

4. Dispositif d'anesthésie suivant l'une quelconque des revendications précédentes, **caractérisé par** un débitmètre (34 ; 70) monté dans une ligne (10 ; 58) d'inspiration du circuit (10, 14, 24 ; 58, 60, 66) de respiration afin de déterminer une valeur du débit instantané de gaz de respiration, l'unité (18 ; 64) de commande régulant le refoulement du produit liquide anesthésique par la micropompe (46) en fonction de la valeur instantanée du débit déterminée par le débitmètre (34 ; 70) et d'une valeur de référence définie à l'avance correspondant à la concentration désirée de produit anesthésique dans le gaz de respiration.

5. Dispositif d'anesthésie suivant l'une quelconque des revendications précédentes, **caractérisé par** un premier thermomètre (30) monté dans le circuit (10, 14, 24) de respiration et destiné à repérer la température du gaz de respiration, par un deuxième thermomètre (40A) monté dans le récipient (38A) et destiné à repérer la température du produit anesthétique liquide, et un manomètre (32) est monté dans le circuit (10, 14, 24) de respiration pour mesurer la pression du gaz de respiration, l'unité (18) de commande commandant alors le refoulement de produit anesthésique liquide par la micropompe (46) suivant les températures et pression mesurées.

6. Dispositif d'anesthésie suivant l'une quelconque des revendications précédentes, **caractérisé par** un moyen (20) pouvant être chauffé formant évaporateur qui est monté dans le moyen (14) de liaison pour évaporer du produit anesthésique liquide de l'unité (16) de dosage.

7. Dispositif d'anesthésie suivant la revendication 6, **caractérisé en ce que** l'unité (18) de commande est reliée au moyen (20) formant évaporateur pour réguler la température du moyen, la température dépendant alors des propriétés d'évaporation du produit anesthésique liquide.

8. Dispositif d'anesthésie suivant l'une quelconque des revendications précédentes, **caractérisé en ce que** l'unité (16) de dosage comprend au moins un récipient (38B, 38C, 38D, 38D) supplémentaire contenant un produit anesthésique liquide supplémentaire, et le récipient (38A) et le récipient (38B, 38C, 38D, 38E) supplémentaire communiquent avec la micropompe (46) par une unité (14) à vanne conçue de manière à ce que seulement l'un des récipients (38A, 38B, 38C, 38D, 38E) à la fois puisse relâcher du produit anesthésique liquide vers la micropompe (46).

9. Dispositif d'anesthésie suivant l'une quelconque des revendications précédentes, **caractérisé par** un dispositif de mesure (36) du gaz, monté sur le circuit (10, 14, 24) de respiration et destiné à mesurer la concentration du produit anesthésique dans le gaz de respiration, l'unité (18) de commande commandant alors la micropompe (46) en fonction de la concentration du produit anesthésique qui est mesurée.
